(19) Europäisches Patentamt
European Patent Office
Office européen des brevets

(11) Publication number: **0 004 390**
**A1**

# (12) EUROPEAN PATENT APPLICATION

(21) Application number: 79100961.6

(22) Date of filing: 29.03.79

(51) Int. Cl.²: C 07 C 69/14, C 07 C 69/24, C 07 C 67/00

(30) Priority: 29.03.78 IT 2171778

(43) Date of publication of application: 03.10.79 Bulletin 79/20

(84) Designated Contracting States: BE CH DE FR GB NL

(71) Applicant: Montedison S.p.A., 31, Foro Buonaparte, Milan (IT)

(72) Inventor: Giordanò, Claudio, 3, Via Costa, Novara (IT)
Inventor: Belli, Aldo, 19, Via Palestro, Intra (Novara) (IT)
Inventor: Minisci, Francesco, 19, Via Marescalchi, Milan (IT)

(74) Representative: Weinhold, Peter, Dr. et al, Siegfriedstrasse 8, D-8000 München 40 (DE)

## (54) Process for preparing alpha-naphthol esters of carboxylic acids and the products thereof.

(57) A process for preparing alpha-naphthol esters of aliphatic carboxylic acids by reaction of naphthalene with aliphatic carboxylic acids, consisting in the reaction of an aliphatic carboxylic acid in a medium consisting of an aliphatic carboxylic acid and in the presence of a system composed of a saline compound of cobalt II, of a ketone containing in its structure at least a methylene or methine group in alpha position in respect of the carbonyl group, and of oxygen, at a temperature ranging from 70° to 110°C.

EP 0 004 390 A1

This invention relates to a process for preparing alpha--naphthol esters of carboxylic acids.
In particular, the present invention relates to a process for preparing alpha-naphthol esters of aliphatic carboxylic acids by oxidation of naphthalene in the presence of cobalt compound(s).

The alpha-naphthol esters so obtained are interesting compounds having a wide field of applications. In fact, they can be usefully employed, besides as useful intermediates for the synthesis of organic compounds in general, also in the field of the dyes and the perfumes - such as, for example, the alpha-naphthol obtainable from the esters by conventional hydrolysis - and of the insecticides, such as the N-methylcarbamate of alpha-naphthol etc.

Since such utilizations substantially aim at preparing the so-called fine chemicals, the purity degree of the starting product is of particular importance or even a decisive requirement for the industrial application.
In the case of alpha-naphthol, for instance, the absence of the beta-naphthol isomer or at least the possibility of reducing it to negligible values, is of importance. On the other hand it is known that the two alpha- and beta-naphthol isomers are in practice difficult to be sufficiently separated by means of the usual industrial methods.

Consequently that involves, the necessity of having recourse to complicated and expensive syntheses to secure a reasonable selectivity as to the desired alpha--naphthol isomer.

For example, alpha-naphthol can be prepared by nitration of naphthalene under controlled conditions, followed by the reduction of the nitrocompound to naphthylamine; the alpha and beta isomers are then separated and finally alpha-naphthol is obtained by acidic hydrolysis at high temperature.

Alpha-naphthol can be prepared also by sulphonation of naphthalene with 98%-sulphuric acid under mild conditions at about 60°C, and by successive alkaline hydrolysis under severe conditions (370°-410°C). It is furthermore obtainable by oxidation of 1-tetralone with sulphur or selenium at high temperatures, or by heterogeneous dehydrogenation with Ni, Pd, Pl.

It is clear that a simpler and more selective method of obtaining alpha-naphthol is of great interest.

Methods of directly oxidizing aromatic compounds in the presence of cobalt compounds are already known.

For example it is known how to obtain aromatic acids (therephthalic acid) by oxidizing in one step alkylbenzenes (p-xylene) in the presence of acetate of cobalt II and of ketonic activators. Oxidation is selectively directed to the alkyl chains on the aromatic nucleus, while the process is of the auto-catalytic type.

Finally, a process has been described for preparing alpha-naphthol esters of aliphatic carboxylic acids by reacting naphthalene in the presence of aliphatic carboxylic acids with organic salts of cobalt III in the absence of gaseous oxygen and in an inert atmosphere, using narrow concentration conditions of cobaltic ions: CoIII/CoII $\geq$ 1. However the yields are not fully satisfactory in respect of the cobaltic salts.

As far as the Applicant knows, no method has been described so far regarding the preparation of alpha-naphthol esters of aliphatic carboxylic acids, which includes the selective and direct introduction of an aliphatic acyloxy group on the naphthalene nucleus, using salts of cobalt II and oxygen.

Thus it is an object of this invention to provide a simple and economic process for preparing alpha-naphthol esters of aliphatic carboxylic acids, which may be free from the drawbacks of the prior art described hereinbefore.

This and still other objects, which will become obvious for those skilled in the art from the following description, are achieved, according to the present invention, by a process for preparing alpha-naphthol esters of aliphatic carboxylic acids, characterized in that naphthalene is reacted with an aliphatic carboxylic acid in a medium consisting of an aliphatic carboxylic acid in the presence of a system consisting of a saline compound of cobalt II, of a ketone containing at least a methylene group or a methine group in alpha position in respect of the carbonyl group, and of oxygen and/or an oxygen-containing gas at a temperature ranging approximately from 70° to 110°C.

The reaction can be schematically represented by the following equation:

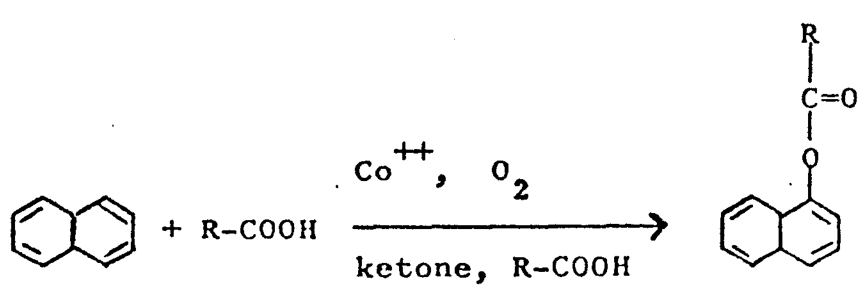

wherein R is an alkyl having up to 10 carbon atoms, and $Co^{++}$ is a Co compound selected from amongst the salts of an organic or an inorganic anion, such as for example salts of aliphatic carboxylic acids, the chloride, the sulphate, the acetylacetonate, etc.
More explicitly, the process consists therefore in reacting naphthalene with an aliphatic carboxylic acid in the presence of a compound of CoII in a medium consisting of an aliphatic carboxylic acid R-COOH, in the presence of an oxidizing system composed by oxygen and/or an oxygen--containing gas and by a ketone.

Such behaviour of naphthalene in the presence of molecular oxygen is all the more surprising as one skilled in the art should have reasonably expected, under the conditions described hereinbefore, an remarkable, if not even a predominant oxidation of naphthalene to secondary compounds (naphthoquinone, phthalic acid, etc.).

According to this invention, oxygen and/or gases containing it, preferably air, are used as oxidizing gas.
Advantageous results are obtained, for example, by using oxygen amounts varying from 60 to 600 liters/hour per mole of the compound of cobalt II, or corresponding amounts of air. The compound of cobalt II, according to this invention, is a salt of an inorganic anion (chloride, sulphate, etc.), of an organic anion derived from a carboxylic acid R-COOH (acetate, propionate, etc.) or an enolate (acetylacetonate, etc.). Preferably it is the salt of the same aliphatic acid R-COOH used as reagent, or mixtures thereof, in which R has the meaning indicated hereinabove.

Advantageous results are attained by using anhydrous and/or hydrated cobaltous acetate, propionate, acetylacetonate, chloride, sulphate etc.

When an inorganic salt of Co II (chloride, etc.) is employed, the reaction is promoted by the presence of alkaline acetates or of buffering agents in general.
The compound of cobalt II is utilized in an initial molar ratio of the compound of cobalt II to naphthalene approximately ranging from 10:1 to 1:10, preferably the molar ratio ranges from about 2:1 to about 1:2.
The initial concentration of the compound of cobalt II is advantageously adjusted to an initial value varying from 0.2 to 0.5 moles of Co II per liter of reacting mass.

The reaction temperature is between about 70° and 110°C, values around 90°C being preferred.

The reaction is conducted in a medium consisting of an aliphatic carboxylic acid of formula R-COOH as defined hereinbefore, preferably it is the same acid which constitutes the salt of cobalt II or mixtures thereof.

Usually atmospheric pressure is used.

The ketone being part of the ketone/oxygen oxidizing system consists of a ketone containing in its structure at least a methylene or methine group ( >CH-) in alpha position in respect of the carbonyl group. The ketone is selected from the aliphatic, cycloaliphatic, aliphatic-aromatic ketones.

The following ketones have proved to be effective: ethylmethylketone, diethylketone, cyclohexanone, propiophenone, methylisopropylketone.

They are introduced in a molar ratio to the compound of Co II ranging from about 4:1 to 8:1.

After an induction period which varies as a function of the temperature etc. from about 30 minutes to 1 hour, the reaction starts in a moderately exothermic way and terminates in about 2-6 hours.

According to an effective embodiment of this invention, the process is conducted as follows.

The salt (acetate) of cobalt II, the naphthalene and the aliphatic carboxylic acid (acetic acid) are introduced, in the desired ratios into a reactor equipped with a stirrer, thermometer, cooler, charging and gas introducing systems. Successively oxygen is made to bubble under stirring, then the mixture is heated to about 90°C and the ketone (ethylmethyl ketone) is introduced. After an initial induction time (from 45 minutes to 1 hour), a moderately exothermic reaction is started and the temperature is adjusted at 90-93°C for a total about 4 hours. After cooling to room temperature, the naphthol ester (acetate) is separated by extraction, and is washed etc. according to conventional methods.

The process appears particularly advantageous, due to the mild and simple operating conditions.

A further advantage consists in the high selectivity and in the obtainment exclusively of an alpha-naphthol having such a purity degree as to allow the direct use of same without further purifications from the beta-naphthol isomer.

The present invention is described in more detail by the following examples, which are given, however, for illustrative purposes only.
For comparative purposes example 14 was conducted in the absence of ketones in the catalytic system.

EXAMPLE 1

In a reactor consisting of a 250-ml flask equipped with a mechanical agitator, a thermometer, a cooler, a plunging pipe for gas bubbling and a feeding funnel, 2-butanone (36 g; 0.5 moles) was added in 5 minutes to a solution of $Co\,(OCCH_3)_2 \cdot 4H_2O$ (14.94 g; 0.06 moles) of naphthalene (10.24 g; 0.08 moles) in glacial acetic acid (150 ml), the temperature was maintained at $90^{o}C$, under intense stirring (1100 rpm) in an oxygen stream (10 l/h).
The reaction mixture was then kept at $90^{o}C$, under strong intense stirring and in an oxygen stream for total 4 hours. During the first two hours the color of the solution changed from violet to dark green; this stage was accompanied by a slight exothermicity of the reaction.
The reaction mass was cooled to room temperature, was poured into water and extracted with ethyl ether. The ethereal extracts were gathered and washed at first with water and then with a sodium bicarbonate-saturated solution, whereupon they were dried.
By gas-chromatographic analysis of the reaction raw product so obtained, it was possible to determine that 19.6% of the fed naphthalene was converted to alpha-acetoxy-naphthalene. The gas-chromatographic and nuclear magnetic resonance analyses carried out on the reaction raw product revealed that β-acetoxy-naphthalene was absent.
2.26 g of α-naphthol ($15.7 \times 10^{-3}$ moles) were obtained by hydrolysis of the reaction raw product.

EXAMPLE 2

The same procedure was followed as described in example 1, but using a reduced amount of naphthalene (5.12 g; 0.04 moles) for a shorter reaction time (2 hours and 30 minutes). The gas-chromatographic analysis of the reaction raw product revealed that 33.7% of the fed naphthalene was converted to α-acetoxy-naphthalene. β-acetoxy-naphthalene was absent.

BAD ORIGINAL

EXAMPLE 3

The same procedure was followed as described in example 1, but using a reduced amount of 2-butanone (18 g; 0.25 moles). From the gas-chromatographic analysis (G.L.C.) of the reaction raw product it appeared that 18.5% of the fed naphthalene was converted to α-acetoxy-naphthalene. β-acetoxy-naphthalene was absent.

EXAMPLE 4

The same procedure was followed as described in example 1, but using a reduced amount of 2-butanone (9 g; 0.125 moles). The G.L.C. analysis of the reaction raw product revealed that 11% of the fed naphthalene was converted to α-acetoxy-naphthalene. β-acetoxy-naphthalene was absent.

EXAMPLE 5

The same procedure was followed as described in example 1, but at a higher temperature (105°C) and with a shorter reaction time (3 hours).
The G.L.C. analysis of the reaction raw product revealed that 10% of the fed naphthalene was converted to α-acetoxy-naphthalene.

EXAMPLE 6

The same procedure was followed as described in example 1, but employing a higher oxygen flow (20 l/h).
The G.L.C. analysis of the reaction raw product shows that 21.3% of the fed naphthalene was converted to α-acetoxy-naphthalene.

EXAMPLE 7

The same procedure was followed as described in example 1, but using a lower oxygen flow (5 liters/hour).
From the G.L.C. analysis of the reaction raw product it appeared that 14.4% of the fed naphthalene was converted to α-acetoxy-naphthalene.

EXAMPLE 8

The same procedure was followed as described in example 1, but using 3-pentanone (42 g; 0.5 moles) instead of 2-butanone, and employing a shorter reaction time (3 hours and 30 minutes).
The G.L.C. analysis of the reaction raw product revealed that 29% of the fed naphthalene was converted to α-acetoxy-naphthalene.

EXAMPLE 9

The same procedure was followed as described in example 1, but using phenylethylketone (67 g; 0.5 moles) instead of 2-butanone and employing a longer reaction time (5 hours and 30 minutes).
From the G.L.C. analysis of the reaction raw product it appeared that 14% of the fed naphthalene was converted to α-acetoxy-naphthalene.

EXAMPLE 10

The same procedure was followed as described in example 1, but using cyclohexanone (23.5 g 0.24 moles) instead of 2-butanone, employing a lower temperature (70°C) and a shorter reaction time (3 hours).
The G.L.C. analysis of the reaction raw product revealed that 3% of the fed naphthalene was converted to α-acetoxy-naphthalene.

EXAMPLE 11

The same procedure was followed as described in example 1, but using $Co(O\overset{O}{\overset{\|}{C}}CH_3)_2$ (10.62 g; 0.06 moles) instead of $Co(O\overset{O}{\overset{\|}{C}}CH_3)_2 \cdot 4H_2O$ and employing a longer reaction time (4 hours and 30 minutes).
From the G.L.C. analysis of the reaction raw product it resulted that 23.5% of the fed naphthalene was converted to α-acetoxy-naphthalene.

BAD ORIGINAL

EXAMPLE 12

The same procedure was followed as described in example 1, but using propionic acid (150 ml) instead of acetic acid. The G.L.C. analysis of the reaction raw product revealed that 2.2% of the fed naphtalene was converted to α-naphthyl-propionate.

EXAMPLE 13

The same procedure was followed as described in example 1, but using 3-methyl-2-butanone (43 g; 0.5 moles) instead of 2-butanone, and employing a lower temperature (20°C) and a longer reaction time (9 hours and 30 minutes).
The G.L.C. analysis of the reaction raw product revealed that 6.2% of the fed naphthalene was converted to α-acetoxy-naphthalene.

EXAMPLE 14 (comparative test)

By way of comparison the same procedure was followed as described in example 1, but in the absence of ketones and for a longer reaction time (8 hours).
The G.L.C. analysis of the reaction raw product revealed that less than 1% of the fed naphthalene was converted to α-acetoxy-naphthalene.

EXAMPLE 15

The same procedure was followed as described in example 1, but using air instead of oxygen and with a higher flow (30 liters/hour).
From the G.L.C. analysis of the reaction raw product it appeared that 5.5% of the fed naphthalene was converted to α-acetoxy-naphthalene.

EXAMPLE 16

The same procedure was followed as described in example 1, but using $CoCl_2.6H_2O$ instead of $Co(O\underset{\underset{O}{\|}}{C}CH_3)_2.4H_2O$. The G.L.C. analysis of the reaction raw product revealed that 3.5% of the fed naphthalene was converted to α-acetoxy-naphthalene.

EXAMPLE 17

The same procedure was followed as described in example 1 in the presence of potassium acetate (11.76 g; 0.12 moles). The G.L.C. analysis of the reaction raw product revealed that 13% of the fed naphthalene was converted to -acetoxy-α-naphthalene.

BAD ORIGINAL

0004390

WHAT WE CLAIM IS:

1) A process for preparing alpha-naphtol esters of aliphatic carboxylic acids by reaction of naphthalene with aliphatic carboxylic acids, characterized in that naphthalene is reacted with an aliphatic carboxylic acid in a medium consisting of an aliphatic carboxylic acid and in the presence of a system composed of a saline compound of cobalt II, of a ketone containing in its structure at least a methylene or methine group in alpha position in respect of the carbonyl group, and of oxygen, at a temperature ranging from 70° to 110°C.

2) A process according to claim 1, characterized in that the compound of cobalt II is a salt of an inorganic anion or of an organic anion derived from an aliphatic carboxylic acid R-COOH, wherein R is an alkyl having up to 10 carbon atoms, or is a cobalt enolate.

3) A process according to claims 1 and 2, characterized in that the medium is an aliphatic carboxylic acid R-COOH, wherein R is an alkyl having up to 10 carbon atoms, and preferably is the same acid on which the salt of cobalt II is based.

4) A process according to any of the preceding claims, characterized in that air is used as a source of oxygen.

5) A process according to any of the preceding claims, characterized in that the compound of cobalt II is selected from amongst the anhydrous or hydrated acetate, propionate, chloride, sulphate, acetylacetonate.

6) A process according to any of the preceding claims, characterized in that the compound of cobalt II is utilized in an initial molar ratio with respect to naphthalene approximately ranging from 10:1 to 1:10, preferably in a ratio approximately ranging from 2:1 to 1:2.

7) A process according to any of the preceding claims, characterized in that the initial concentration of the compound of cobalt II is between about 0.2 and 0.5 moles/liter.

8) A process according to any of the preceding claims, characterized in that the temperature is maintained at about 90°C.

9) A process according to any of the preceding claims, characterized in that the oxygen flow and/or the flow of the gas containing it ranges from 60 to 600 liter/hour of $O_2$ per mole of cobalt II.

10) A process according to any of the preceding claims, characterized in that the ketone containing in its structure a methylene or methine group in alpha position in respect of the carbonyl group is selected from the aliphatic, cycloaliphatic, aliphatic-aromatic ketones preferably from ethylmethyl ketone, diethyl ketone, cyclohexanone, propiophenone, methylisopropyl ketone.

11) A process according to any of the preceding claims, characterized in that the ketone is introduced in a molar ratio to the initial cobalt compound ranging approximately from 4:1 to 8:1.

BAD ORIGINAL

12) A process according to any of the preceding claims, characterized in that the inorganic salt of Co II is employed in combination with buffering agents, preferably sodium or potassium acetate.

13) A process for preparing naphtol esters of aliphatic carboxylic acids, as described and exemplified hereinabove.

14) Naphthol esters of aliphatic carboxylic acids, when obtained according to the process as described and claimed hereinbefore.

BAD ORIGINAL

European Patent Office

# EUROPEAN SEARCH REPORT

0004390

Application number

EP 79 10 0961

| Category | DOCUMENTS CONSIDERED TO BE RELEVANT<br>Citation of document with indication, where appropriate, of relevant passages | Relevant to claim | CLASSIFICATION OF THE APPLICATION (Int. Cl.²) |
|---|---|---|---|
| A | FR - A - 2 193 811 (LABOFINA)<br>* Page 1, line 35 - page 2, line 10 *<br>-- | | C 07 C 69/14<br>69/24<br>67/00 |
| A | FR - A - 2 103 890 (ASAHI KASEI)<br>* Page 2, lines 19-40; page 11, example 12 *<br>---- | | |

TECHNICAL FIELDS SEARCHED (Int.Cl.²)

C 07 C 69/14
69/24
67/00

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 21-06-1979 | KINZINGER |

EPO Form 1503.1 06.78